# EUROPEAN PATENT APPLICATION

(11) **EP 3 696 261 A1**
(43) Date of publication of application: **19.08.2020**
(21) Application number: 20154814.6
(22) Date of filing: 03.12.2008
(51) Int. Cl.: C12N 5/0775

(54) **METHODS AND COMPOSITIONS FOR MODULATING DIFFERENTIATION OF PLURIPOTENTIAL CELLS**

(30) Priority: 03.12.2007 US 5211 P; 30.04.2008 US 125941 P; 10.06.2008 US 131577 P
(62) Divisional of application: 15185035.1
(71) Applicant: SanBio, Inc., Mountain View, CA 94041-1522 (US)
(72) Inventor: AIZMAN, Irina, Mountain View, CA 94041 (US)
(74) Representative: Barker Brettell LLP

(57) **Abstract**

Disclosed herein are methods and compositions useful for altering the differentiation potential of marrow adherent stromal cells, also known as mesenchymal stem cells. In certain embodiments, the normal tendency for these cells to differentiate into osteogenic and adipogenic lineages is restricted.

## Description

### CROSS REFERENCE TO RELATED APPLICATIONS

This application is related to United States provisional patent application No. 61/005,211 filed December 3, 2007, to United States Provisional patent application No. 61/125,941, filed April 30, 2008, and to United States Provisional patent application No. 61/131,577, filed June 10, 2008; the disclosures of which are incorporated by reference in their entireties for all purposes, in particular for the purpose of providing color photographs showing the results of osteogenesis and adipogenesis assays.

### STATEMENT REGARDING FEDERAL SUPPORT

Not applicable.

### FIELD

The present disclosure is in the fields of developmental biology and cell therapy.

### BACKGROUND

Mesenchymal stem cells, also known as marrow adherent stromal cells are, as their name suggests, a class of pluripotential cells, found in bone marrow, that can give rise to a number of different connective tissue cell types including,, e.g., osteocytes, chondrocytes, adipocytes, endothelial cells, fibroblasts and smooth muscle cells.

Mesenchymal stem cells can be isolated and purified from bone marrow specimens by virtue of their adherence to the plastic surface of culture vessels. In this way, enriched populations of mesenchymal stem cells, that are essentially free of hematopoietic progenitor cells and marrow stroma, can be obtained from adult bone marrow. A. J. Friedenstein et al. (1976) Exp. Hematol. 4:276 (1976). Such enriched populations of mesenchymal stem cells can be induced, by choice of culture conditions, to differentiate into various types of connective tissue, including bone, cartilage and adipose tissue. See, *e.g.,* Prockop (1997) Science 276:71-74 and Pittenger et al. (1999) Science 284:143-147.

Mesenchymal stem cells do not, in the course of normal ontogeny, develop into cells of the nervous system. However, several methods of treating mesenchymal stem cells in culture have been shown to shift their developmental potential at least partly toward neuronal and glial cells. See, for example, Dezawa et al. (2004) J. Clin. Invest. 113:1701-1710; United States Patents 6,528,245 (March 4, 2003), 6,989,271 (Jan. 24, 2006) and 7,129,034 (Oct. 31, 2006); United States Patent Application Publications 2003/0003090 (Jan. 2, 2003), 2003/0203484 (Oct. 30, 2003), 2004/0235165 (Nov. 25, 2004), 2006/0166362 (July 27, 2006) and 2006/0251624 (Nov. 9, 2006). The disclosures of all of the above-cited documents are incorporated by reference for the purpose of providing methods of converting mesenchymal stem cells to cells having neurogenic potential.

Certain of the aforementioned methods for shifting the developmental potential of mesenchymal stem cells toward neuronal cells involve manipulation of the Notch signaling system. In one aspect of Notch signaling, a first cell that has committed to differentiate into a neuronal lineage signals an adjacent second cell, containing the Notch transmembrane receptor on its surface, so as to prevent differentiation of the second cell into a neuronal lineage. The signal is transmitted *via* a Notch Ligand (*e.g.,* Delta, Serrate, Jagged) on the surface of the first cell and results in cleavage of the Notch protein and translocation of its intracellular domain to the nucleus of the second cell. For a review, see Artavanis-Tsakonas et al. (1999) Science 284:770-776, the disclosure of which is incorporated by reference for the purpose of disclosing aspects of the Notch signaling system.

Thus, in normal ontogeny, cleavage of the Notch protein and release of its intracellular domain from the interior of the surface of a cell can block neural differentiation of that cell. However, transfection of a plasmid encoding the Notch intracellular domain into mesenchymal stem cells (also known as marrow adherent stromal cells) can cause descendents of the transfected cells to develop into neural and myogenic lineages. Dezawa et al. (2004) J. Clin. Invest. 113:1701-1710; and United States patent application publication No. 2006/0166362 (July 27, 2006), the disclosures of which are incorporated by reference for the purpose of describing methods of inducing mesenchymal stem cells to develop into cells having neurogenic and myogenic potential by transfecting mesenchymal stem cells with a nucleic acid encoding a Notch intracellular domain.

Many details of the processes, mentioned above, by which the differentiation capacity of mesenchymal stem cells is re-directed remain unknown. For example, the mechanism by which transfection of sequences encoding the Notch intracellular domain shifts the developmental potential of mesenchymal stem cells is not understood. Furthermore, the potential of mesenchymal stem cells to retain the ability to differentiate into their normal descendents (e.g., adipocytes, osteocytes, chondrocytes), after they have undergone treatments that alter their developmental repertoire to include neural and myogenic lineages, has not been determined. A greater understanding of these question has important implication for the potential therapeutic use of mesenchymal stem cells and their derivatives in areas such as neurodegenerative disorders, musculoskeletal disorders, cartilage repair and wound healing.

### SUMMARY

It has now been determined, and is disclosed herein, that treatments which alter the developmental potential of marrow adherent stromal cells (MASCs, also known as mesenchymal stem cells) by allowing them to differentiate into non-mesenchymal lineages also restrict the ability of such treated cells to differentiate into cells of the mesenchymal lineage. That is, the normal developmental potential of marrow adherent stromal cells has been both expanded and reduced.

Accordingly, disclosed herein are methods and compositions for redirecting the developmental potential of marrow adherent stromal cells. Also provided are marrow adherent stromal cells and their descendents with redirected developmental potential, as exemplified by the following embodiments.
**1.** A method for altering the developmental potential of a marrow adherent stromal cell, the method comprising:
   (a) providing a marrow adherent stromal cell; and
   (b) transfecting the cell with a nucleic acid comprising sequences encoding a Notch intracellular domain (NICD).
   In certain embodiments transfection is transient, such that the transfected nucleic acid does not persist in descendants of the transfected cell. This can result in transient expression of the NICD.
   In additional embodiments, stable expression of a NICD, for a limited amount of time, occurs in the transfected cells. That is, transfected cells are placed under selection for the nucleic acid encoding the NICD, but selection is withdrawn after a predetermined amount of time (*e.g.,* one or more days, one week, several weeks).
**2.** The method according to embodiment 1, wherein development of the marrow adherent stromal cell into an osteogenic lineage is reduced.
**3.** The method according to embodiment 1, wherein development of the marrow adherent stromal cell into an adipogenic lineage is reduced.
**4.** The method according to embodiment 1, wherein development of the marrow adherent stromal cell into both osteogenic and adipogenic lineages is reduced.
**5.** The method according to embodiment 1, wherein the NICD is a human NICD.
**6.** The method according to embodiment 5, wherein the NICD comprises amino acids 1703-2504 of the human Notch protein.

### BRIEF DESCRIPTION OF THE DRAWINGS

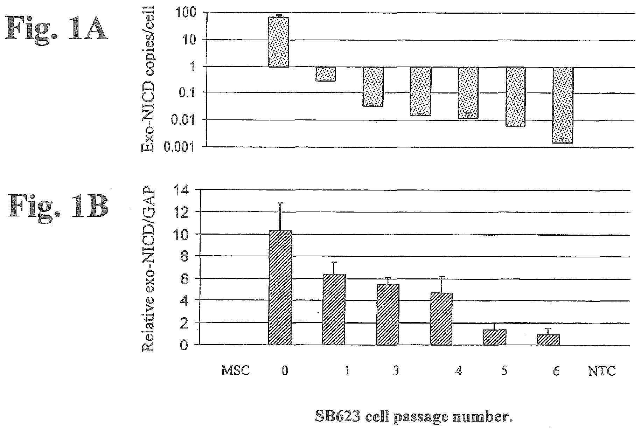

**Figures 1A and 1B** show levels of NICD-encoding DNA (Figure 1A) and mRNA (Figure 1B) in differentiation restricted cells (DRCs) through six passages. Figure 1A shows copies per cell of exogenous, transfected NICD-encoding sequences for passages 0 through 6. Figure 1B shows levels of mRNA transcribed from the exogenous, transfected NICD-encoding sequences, normalized to GAPDH mRNA levels. Data for Passage 0 shows DNA and RNA levels after one week of selection in G418 followed by two weeks in culture after removal of G418. "MSC" denotes untransfected marrow adherent stromal cells. "NTC" denotes non-template control; *i.e.,* an amplification reaction conducted with all components except cellular DNA or RNA.

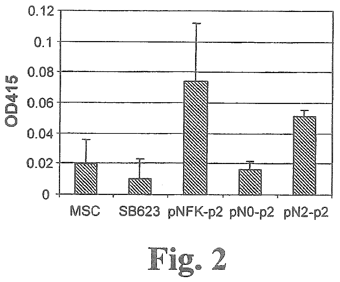

**Figure 2** shows levels of Alizarin Red extracted from cells that had been transfected with different NICD-encoding vectors and controls. Cells were transfected with the indicated vector, grown for two passages in the presence of 100 µg/ml G418 (except for DRCs, labeled "SB623" in the Figure), then cultured in osteogenic differentiation medium (including 500 ng/ml BMP6) for six days. Cells were fixed, stained and Alizarin Red was extracted and quantitated by measuring absorbance at 450 nm. See Example 9 for details.

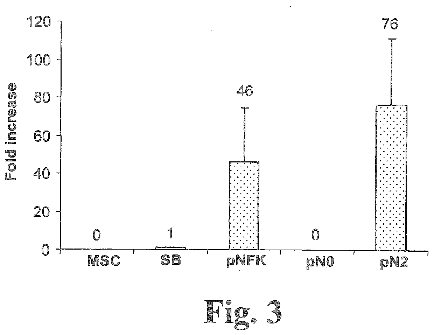

**Figure 3** shows levels of NICD mRNA in MASCs that had been transfected with different vectors, selected in 100 µg/ml G418 for two passages, then cultured in for an additional three days in the presence of G418. Control MASCs were not transfected and not subjected to selection ("MSC"). DRCs (indicated by "SB" in the figure) were assayed after transfection, selection for one week in G418, and three passages. Levels of RNA transcribed from the NICD-encoding sequences in the vector were normalized to endogenous levels of GAPDH mRNA in the cells. Results are expressed relative to this normalized value in DRCs.

### DETAILED DESCRIPTION

The terms "bone marrow stromal cells," "marrow adherent stromal cells," "marrow adherent stem cells," "marrow stem cells," "mesenchymal stem cells" and "MASCs" refer to mitotic, pluripotent cells, obtained from bone marrow that, in the course of normal development, are capable of giving rise to a number of differentiated cell types such as, for example, osteocytes, and cells normally found in connective tissue including, but not limited to, chondrocytes and adipocytes. MASCs can be human cells or cells from other mammals or vertebrates.

The terms "neural precursor cell," "neural progenitor cell, "NPC" and "bone marrow-derived neural progenitor cell" are used interchangeably to refer to mitotic cells, descended from marrow adherent stromal cells, that have the capability to differentiate into neurons, glial cells, or their precursors. They are thus distinct from primary neural precursor cells, such as can be obtained from fetuses or adult tissues such as the hippocampus and the periventricular subependymal zone. NPCs can be human cells or cells from other mammals or vertebrates.

For the purposes of this disclosure, the terms "differentiation-restricted cell" and "DRC" refer to a cell, descended from a mesenchymal stem cell, whose normal lineage specification (to mesenchymal lineages such as osteocytes, chondrocytes and adipocytes) has been restricted. That is, the ability of such a cell to enter a lineage resulting in terminal differentiation into osteocytes, adipocytes and/or chondrocytes is reduced. A differentiation restricted cell may optionally acquire one or more new developmental potentials including but not limited to the ability to enter a neural lineage and differentiate into neurons and/or glial cells, and/or the ability to enter a myogenic lineage. A differentiation restricted cell may also acquire the ability, or gain an enhanced ability, to secrete soluble and/or insoluble factors that promote regeneration and/or growth of neural tissue (*e.g*., neurons, astrocytes, oligodendrocytes, microglia, Schwann cells), and/or prevent death of neural tissue.

The present disclosure provides methods and compositions for, *inter alia,* restricting the differentiation potential of marrow adherent stromal cells. That is, the normal ontogenic potential of marrow stromal cells to enter mesenchymal lineages and give rise to, *e.g*., chondrocytes, osteocytes and adipocytes, is restricted; and their development can be redirected into other lineages, *e.g*., neural and myogenic, making them useful for various types of cell therapy, *e.g*., in the treatment of neural degeneration, stroke, muscular dystrophy, *etc.*

Marrow adherent stromal cells are easily extracted by bone marrow aspiration on an outpatient basis, and due to their highly proliferative nature they can be cultured in large amounts within a relatively short period. Moreover, a further advantage of their use as starting material for various types of cell therapy is that they allow autologous transplantation to be carried out (*e.g.,* new muscular and/or neural tissue can be formed from cells derived from the patient's own bone marrow stem cells). The consequent lack of immunological rejection dispenses with the need for administering immunosuppressants, thus enabling safer treatment. Furthermore, since bone marrow stem cells can be obtained from a bone marrow bank, this method is also advantageous from a supply standpoint.

Marrow adherent stromal cells can be obtained from bone marrow aspirates by culturing for three days in α-MEM + 10% FBS + L-Glutamine, then aspirating away non-adherent cells. See Example 1 below for an exemplary method for extraction and expansion of marrow adherent stromal cells.

In certain embodiments, differentiation-restricted cells are obtained by methods comprising transfection of marrow adherent stromal cells with a polynucleotide comprising a sequence encoding a Notch intracellular domain (NICD) as described, for example, in US Patent Application Publication No. 2006-0166362 (July 27, 2006), and Dezawa et al. (2004) J. Clin. Invest. 113:1701-1710; the disclosures of which are incorporated by reference. In certain of these embodiments, the Notch intracellular domain consists of amino acids 1703-2504 of the human Notch-1 protein. Ellison et al. (1991) Cell 66:649-661. In additional embodiments, differentiation-restricted cells are obtained as described in US Patent Application Publication No. 2006-0251624 (November 9, 2006), the disclosure of which is incorporated by reference. Alternatively, such cells can be obtained as described in US Patent Application Publication No. 2003-0003090 (January 2, 2003), the disclosure of which is incorporated by reference. Example 2 describes an exemplary method for the preparation of DRCs.

The methods and compositions disclosed herein involve the use of art-recognized procedures in molecular biology, cell biology and cell culture. Such methods are known to those of skill in the art and have been disclosed, *e.g.,* in Sambrook et al. "Molecular Cloning: A Laboratory Manual," Third Edition, Cold Spring Harbor Laboratory Press, 2001; Ausubel et al., "Current Protocols in Molecular Biology," John Wiley & Sons, New York, 1987 and periodic updates; and R. I. Freshney "Culture of Animal Cells: A Manual of Basic Technique," Fifth Edition, Wiley, New York, 2005.

Certain of the examples show that treatments of MASCs (obtained from two different donors) that result in their ability to differentiate into non-mesenchymal lineages also restrict the ability of such treated cells to achieve their normal developmental potential (*i.e.,* such treatments restrict their ability to differentiate into mesenchymal cells such as osteocytes and adipocytes). MASCS obtained from six additional donors have been tested similarly and, in all 8 cases, treated cells consistently showed a reduced ability, compared to MASCs, to differentiate into adipocytes and osteocytes.

This restricted developmental potential of these "differentiation-restricted cells (DRCs)" encourages the use of DRCs and their descendents in various types of cellular therapies. For example, DRCs can be induced to differentiate into neural cells and neural cell precursors under appropriate culture conditions. See, for example, Dezawa et al. (2004) J. Clin. Invest. 113:1701-1710 and US Patent Application Publication No. 2006-0166362 (July 27, 2006). Use of such neural cells and neural precursor cells for treatment of neural injuries, by transplantation into areas where they may potentially be exposed to osteogenic and/or adipogenic signals (*e.g*., around injuries of the peripheral nervous system) is preferable to the use of MASCs, since the ability of the former cells type to differentiate into osteocytic and adipocytic lineages is reduced or eliminated. Similarly, for use in cell therapeutics that are transplanted into the brain or central nervous system, the shift in developmental potential away from mesenchymal lineages increases the safety and efficacy of DRCs, compared to MASCs and related cell types. Thus, DRCs have a better safety profile than other types of cells that can be used for transplantation.

Certain of the examples show that regulation of gene expression is altered in differentiation-restricted cells. In particular, levels of mRNA encoded by the Hey1 and cyclin D1 genes are increased; while levels of Hes1, HR and Wisp1 mRNAs are decreased, in DRCs compared to their MASC progenitors. Thus, DRCs are distinguished, from their MASC progenitors and from other cells, by virtue of this differential gene expression. Hence, methods of artificially up-regulating Hey 1 and/or cyclin D1 expression, and/or artificially down-regulating Hes1, HR and/or Wisp1 expression, can also be used to generate differentiation-restricted cells. For example, as is known in the art, gene expression can be up-regulated by inserting a cDNA encoding the gene product into a cell; and gene expression can be down-regulated by inserting siRNA, shRNA, micro RNA and/or antisense RNA, complementary to the gene of interest, into a cell. In addition, artificial transcription factors can be used for both activation and repression of gene expression in a cell. See, for example, U.S. Patents 6,534,261 and 6,933,113; the disclosures of which are incorporated by reference for the purpose of describing the synthesis and uses of artificial transcription factors.

### EXAMPLES

### Example 1: Preparation of Marrow Adherent Stromal Cells (MASCs)

Bone marrow aspirates, obtained from human donors, were divided into 12.5 ml aliquots in 50 ml tubes, and 12.5 ml of growth medium (10% FBS in αMEM, supplemented with penicillin/streptomycin and 2 mM L-glutamine) was added to each tube. The contents of the tubes were mixed by inversion and the tubes were centrifuged at 200 x g for 8 minutes. The upper, clear phase was discarded, the volume of the lower phase was adjusted to 25 ml with fresh growth medium, and the tubes were again mixed and centrifuged. The upper layer was again removed. The volume of the lower phase in each tube was again adjusted to 25 ml and the contents of all tubes was pooled in a 250 ml tube. After determination of cell concentration by Trypan Blue exclusion and determination of nucleated cell count, cells were plated in T225 flasks, in 40 ml per flask of growth medium at a density of 100 x 10⁶ total nucleated cells per flask. The flasks were incubated at 37°C for 3 days in a CO₂ incubator, during which time the MASCs attached to the flask.

After 3 days, unattached cells were removed by rocking the flasks and withdrawing the culture medium. Each flask was washed three times with 40 ml of αMEM supplemented with penicillin/streptomycin; then 40 ml of prewarmed (37°C) growth medium was added to each flask and the cells were cultured at 37°C in a CO₂ incubator. During this time, the medium was replaced with 40 ml of fresh growth medium every 3-4 days, and cells were monitored for growth of colonies and cell density.

When the cultures achieved 25-30% confluence (usually 10,000- 20,000 cells per colony and within 10-14 days), the MASCs (passage M0) were harvested for further passage. MASCs were harvested from up to 10 T-225 flasks at a time. Medium was removed from the flasks and the adherent cells were rinsed with 20ml of DPBS w/o Ca/Mg (DPBS -/-, HyClone) 2 times. Ten ml of 0.25% Trypsin/EDTA (Invitrogen, Carlsbad, CA) was added to each flask and flasks were incubated for approximately 5 min at room temperature. When cells had detached and the colonies had dispersed into single cells, the trypsin was inactivated by addition of 10ml of growth medium followed by gentle mixing. The cell suspensions were withdrawn from the flasks, and pooled in 250ml tubes. The tubes were subjected to centrifugation at 200 x *g* for 8 minutes. The supernatants were carefully removed and the wet cell pellets were resuspended in growth medium to an estimated cell concentration of approximately 1x10⁶ cells/ml. Viable cell count was determined and cells were plated in T225 flasks at a concentration of 2 x 10⁶ cells per flask in growth medium (passage M1). Cells were grown for 3-5 days, or until 85-90% confluent, changing medium every 2 to 3 days. At 85-90% confluence, passage M1 cells were harvested by trypsinization and replated at 2 x 10⁶ cells per T225flask as described above, to generate passage M2 cultures. M2 cultures were fed fresh medium every three days, if necessary. When passage M2 cultures reached 85-90% confluence (usually within 3-5 days), they were either harvested for transfection to generate DRCs (Example 2 below) or frozen for future use (Example 3 below).

### Example 2: Preparation of Differentiation-Restricted Cells (DRCs)

DRCs were made either directly from MASCs harvested from passage M2 cultures, or from passage M2 MASCs that had been frozen as described in Example 3, and thawed and revived as described in Example 4.

### A. Preparation of transfection mixture

Differentiation-restricted cells were made by transfection of passage M2 MASCs with a plasmid encoding the Notch intracellular domain. The plasmid (pN2) comprised a pCI-neo backbone (Promega, Madison, WI) in which sequences encoding amino acids 1703-2504 of the human Notch-1 protein, which encode the intracellular domain, were introduced into the multiple cloning site. In this vector, sequences encoding the NICD are under the transcriptional control of the CMV promoter, a strong constitutive promoter. pN2 also contains a neomycin phosphotransferase gene (which encodes G418 resistance) under the transcriptional control of the SV40 early promoter and enhancer.

For each flask of MASCs, 5 ml of transfection mixture, containing 40µg of plasmid and 0.2 ml of Fugene 6® solution, was used. To make the transfection mixture, the appropriate amount of Fugene® solution (depending on the number of flasks of cells to be transfected) was added to αMEM in a sterile 250ml tube, using a glass pipette. The solution was mixed gently and incubated for 5 min at room temperature. The appropriate amount of plasmid DNA was then added dropwise to the Fugene®/αMEM mixture, gently mixed, and incubated for 30 min at room temperature.

Prior to the addition of pCI-Notch DNA to the Fugene®/MEM mixture, 5ml was removed and placed into a 15ml tube to which was added 40ug of pEGFP plasmid. This solution was used to transfect one flask of cells, as a control for transfection efficiency. For certain experiments, "mock DRCs" were also prepared, by transfecting M2 MASCs with a derivative of the pCI-Notch vector from which the Notch-encoding sequences had been removed. All other procedures were identical.

### B. Transfection

For transfection, passage M2 MASCs were harvested by trypsinization (as described in Example 1) and plated at a density of 2.5 x 10⁶ cells in 40 ml of growth medium per T225 flask. When the cells reached 50-70% confluence (usually within 18-24 hours) they were prepared for transfection, by replacing their growth medium with 35 ml per flask of transfection medium (αMEM +10% FBS without penicillin/streptomycin).

Three hours after introduction of transfection medium, 5ml of the transfection mixture (Section A above) was added to each T-225 flask by pipetting directly into the medium, without contacting the growth surface, followed by gentle mixing. A control T-225 flask was transfected with 40µg of pEGFP plasmid, for determination of transfection efficiency.

After incubating cultures at 37°C in transfection medium for 24 hours, the transfection medium was replaced with αMEM +10% FBS + penicillin/streptomycin.

### C. Selection of Transfected Cells

Selection of cells that had incorporated plasmid DNA was begun 48 hrs after transfection by replacing the medium with 40 ml per flask of selection medium (growth medium containing 100 µg/ml G-418). Fresh selection medium was provided 3 days, and again 5 days after selection was begun. After 7 days, selection medium was removed and the cells were fed with 40 ml of growth medium. The cultures were then grown for about 3 weeks (range 18 to 21 days), being re-fed with fresh growth medium every 2-3 days.

Approximately 3 weeks after selection was begun, when surviving cells began to form colonies, cells were harvested. Medium was removed from the flasks using an aspirating pipette and 20 ml of DPBS without Ca²⁺/Mg²⁺, at room temperature, was added to each flask. The culture surface was gently rinsed, the wash solution was removed by aspiration and the rinse step was repeated. Then 10 ml of prewarmed (37°C) 0.25% Trypsin/EDTA was added to each flask, rinsed over the growth surface, and the flasks were incubated for 5-10 min. at room temperature. Cultures were monitored with a microscope to ensure complete detachment of cells. When detachment was complete, trypsin was inactivated by addition of 10ml of growth medium per flask. The mixture was rinsed over the culture surface, mixed by pipetting 4-5 times with a 10ml pipette, and the suspension was transferred into a sterile 50 ml conical centrifuge tube. Cells harvested from several flasks could be pooled in a single tube. If any clumps were present, they were allowed to settle and the suspension was removed to a fresh tube.

The cell suspensions were centrifuged at 800 rpm (200 x g) for 8 min at room temperature. Supernatants were removed by aspiration. Cell pellets were loosened by tapping the tube, about 10 ml of DPBS without Ca²⁺/Mg²⁺ was added to each tube and cells were resuspended by gently pipetting 4-5 times with a 10ml pipette to obtain a uniform suspension.

### D. Expansion of transfected cells

Cell number was determined for the suspension of transformed, selected cells and the cells were plated in T-225 flasks at 2 x 10⁶ cells per flask (providing approximately 30% seeding of viable cells). This culture is denoted M2P1 (passage #1). M2P1 cultures were fed with fresh medium every 2-3 days, and when cells reached 90-95% confluence (usually 4-7 days after passage), they were harvested and replated at 2 x 10⁶ cells per flask to generate passage M2P2. When M2P2 cultures reached 90-95% confluence, they were harvested for cryopreservation (Example 3) or for further assay.

### Example 3: Cryopreservation

MASCs and DRCs were frozen for storage according to the following procedure. MASCs were typically frozen after passage M2, and DRCs were typically frozen after passage M2P2. Processing 4-5 flasks at a time, medium was aspirated from the culture flasks, 10 ml of 0.25% Trypsin/EDTA (at room temperature) was added to each flask, gently rinsed over the culture surface for no longer than 30 sec, and removed by aspirating. Then 10 ml of warmed (37°C) 0.25% Trypsin/EDTA was added to each flask, rinsed over the growth surface, and the flasks were incubated for 5-10 min. at room temperature. Cultures were monitored by microscopic examination to ensure complete detachment of cells.

When detachment was complete, 10 ml of αMEM growth medium was added to each flask, rinsed over the culture surface, and detached cells were mixed by pipetting 4-5 times with a 10 ml pipette. The cell suspension was transferred into a sterile 250 ml conical centrifuge tube, and any large clumps of cells were removed. Cells harvested from 15-20 flasks were pooled into one 250ml tube.

The tube was subjected to centrifugation at 800 rpm (200 x *g*) for 8 min at room temperature. The supernatant was removed by aspirating. The pellet was loosened by tapping the tube, and about 25 ml of DPBS (-/-) was added to each tube. Cells were resuspended by gently pipetting 4-5 times with a 10ml pipette to obtain a uniform suspension. Any clumps in the suspension were removed by pipetting each sample through a sterile 70 µm sieve placed in the neck of a 50 ml tube.

Cell suspensions were pooled in a 250 ml centrifuge tube and any remaining clumps were removed. The final volume was adjusted to 200ml with DPBS (-/-) and the sample was subjected to centrifugation at 800 rpm (200 x *g*) for 8 min at room temperature. The supernatant was removed by aspiration. The cell pellet was loosened by tapping, 20 ml of DPBS (-/-) was added to the tube and cells were resuspended by mixing well and gently pipetting with a 10 ml pipette. The final volume was adjusted with DPBS (-/-) to give an estimated concentration of approximately 0.5- 1.0 x 10⁶ cells/ml, usually about 4-5ml perT225 flask harvested, or about 200ml for a 40-flask harvest.

A viable cell count was conducted on the suspension, which was then subjected to centrifugation at 800 rpm (200 x *g*) for 8 minutes. The supernatant was aspirated, and the cell pellet was resuspended in cold Cryo Stor solution (BioLife Solutions, Bothell, WA) to a concentration of 12 x 10⁶ cells/ml. One ml aliquots were dispensed into vials, which were sealed and placed at 4°C in a Cryo Cooler. Vials were transferred into a CryoMed (Thermo Forma) freezer rack and frozen.

### Example 4: Thawing and recovery

Frozen cells (MASCs or DRCs) were stored in liquid nitrogen. When needed for experiments, they were quick-thawed and cultured as follows. A tube of frozen cells was placed in a 37°C bath until thawed. The thawed cell suspension (1 ml) was immediately placed into 10 ml of growth medium and gently resuspended. The suspension was centrifuged at 200 x *g*, the supernatant was removed, and cells were resuspended in growth medium to an estimated concentration of 10⁶ cells/ml. Live cells were counted by Trypan Blue exclusion and cells were plated at a density of 2 x 10⁶ cells per T225 flask. Cells were cultured at 37°C in a CO₂ incubator for 3-4 days until cell growth resumed. They were then used in the osteogenic and adipogenic differentiation assays described in Examples 5 and 6.

### Example 5: Restriction of osteogenic differentiation potential of marrow adherent stromal cells

For assay of osteogenic differentiation potential, cells (MASCs, DRCs or mock DRCs) were plated at 10⁴ cells/well in 96-well plates in αMEM (Mediatech, Herndon, VA) supplemented with 10% Fetal Bovine Serum (FBS, HyClone, Logan UT) and cultured overnight.

The next day, the culture medium was replaced with DMEM (Mediatech, Herndon, VA) containing high glucose, pyruvate, glutamine and 10% FBS supplemented with 50 µg/ml Ascorbic acid (L-ascorbic acid 2-phosphate, Wako Chemical, Ltd., Japan, added freshly before use), 10 mM β-glycerophosphate (Sigma-Aldrich, St. Louis, MO), and 100 nM Dexamethasone (Sigma-Aldrich, St. Louis, MO). This "osteogenic differentiation medium" was changed every 3-4 days.

Osteogenic differentiation assays were conducted in the absence or presence of recombinant human bone morphogenetic protein 6 (BMP-6; R&D Systems, Minneapolis, MN), at a concentration of 500 ng/ml.

On day 14 or 17 after the transfer of cells into osteogenic differentiation medium, cultures were assayed for the presence of osteocytes by Alizarin Red staining. Cells were fixed by adding 100% methanol to the wells and incubating on ice for 3 min. The wells were then rinsed with water, and 40 mM alizarin red S (Sigma-Aldrich, St. Louis, MO), pH 4.2 was added. After 2 min at room temperature, the wells were rinsed with water, the plate was placed on the stage of a Zeiss Axiovert 40C microscope and cells in the wells were photographed with a Canon PC1049.

The results of these experiments indicated that, in both the presence and absence of BMP-6, MASCs showed a far greater extent of osteogenic differentiation that did DRCs. In addition, it was determined that inhibition of osteogenic differentiation in DRCs is due to the presence of the Notch intracellular domain (NICD) in these cells, inasmuch as MASCs that were transfected with a vector lacking NICD-encoding sequences (mock DRCs) revealed a degree of Alizarin Red staining similar to that of MASCs. In all cases, BMP-6 increased the degree of osteogenic differentiation observed.

### Example 6: Restriction of adipogenic differentiation potential of marrow adherent stromal cells

For assay of adipogenic differentiation potential, cells were plated at 10⁴ cells/well in 96-well plates in αMEM (Mediatech, Herndon, VA) supplemented with 10% Fetal Bovine Serum (FBS, HyClone, Logan UT) and cultured overnight.

The next day, the culture medium was replaced with DMEM (Mediatech, Herndon, VA) containing high glucose, pyruvate, glutamine and 10% FBS supplemented with 1 µM Dexamethasone, 0.5 µM 1-methyl-3 isobutylxanthine, 100 µM indomethacin and 10 µg/ml insulin (all supplements from Sigma-Aldrich, St. Louis, MO). This "adipogenic differentiation medium" was changed every 3-4 days.

Adipogenic differentiation assays were conducted in the absence or presence of recombinant human bone morphogenetic protein 6 (BMP-6; R&D Systems, Minneapolis, MN) at a concentration of 100 ng/ml.

On day 17-21 after transfer of cells into adipogenic differentiation medium, cells were tested for adipocytic differentiation by staining with Oil Red O. Cells were fixed by adding 10% formalin to the wells and incubating for 30-60 min at room temperature. Wells were then washed with 60% isopropanol and allowed to dry completely. Oil Red O working solution was freshly prepared from Oil Red stock solution (3.5 mg/ml in isopropanol) by diluting it 3:2 in water and filtering. Staining was conducted for 10 min; and the wells were then washed extensively with water. After brief counterstaining with Accustain Harris hematoxylin solution (Sigma-Aldrich, St. Louis, MO), the plate was placed on the stage of a Zeiss Axiovert 40C microscope and cells in the wells were photographed with a Canon PC1049.

The results of these experiments indicated that MASCs were capable of differentiating into adipocytes, as evidenced by cells with fat-filled vacuoles stained by Oil Red O, and that adipocytic differentiation of MASCs was enhanced by BMP-6. In contrast, few, if any, adipocytes were formed by DRCs, in either the presence or absence of BMP-6; and those that did form contained much smaller vacuoles. Control experiments showed that inhibition of adipogenic differentiation in DRCs is due to the presence of the Notch intracellular domain (NICD) in these cells, inasmuch as MASCs that were transfected with a vector lacking NICD-encoding sequences (mock DRCs) revealed a degree of Oil Red O staining similar to that of MASCs.

### Example 7: Osteogenic potential of MASCs is enhanced by activation with Jagged, a Notch ligand

The human Jagged1 protein is a ligand of the human Notch1 protein. Thus, a cell expressing Jagged on its surface, will, when contacted with a Notch-expressing cell, induce cleavage of transmembrane Notch and generation of cytoplasmic NICD in the Notch-expressing cell. The effect of sustained Notch activation in MASCs, by the Jagged protein, was investigated in the osteogenic differentiation system described in Example 5.

MASCs (prepared essentially as described in Example 1) were plated at 10⁴ cells/well in 96-well plates in αMEM (Mediatech, Herndon, VA) supplemented with 10% Fetal Bovine Serum (FBS, HyClone, Logan UT) and cultured overnight. The next day, cells were transferred to osteogenic differentiation medium (composition described in Example 5).

During the differentiation procedure, certain batches of cells were subjected to Notch activation *via* the Notch ligand Jagged. A fusion between the extracellular domain of the rat Jagged1 protein and the F_{c} region of human IgG (Jag1/F_{c}) was obtained from R&D Systems (Minneapolis, MN; Catalogue No. 599-JG), and the lyophilized preparation was reconstituted to a concentration of 1 mg/ml in PBS containing 0.1%(w/v) bovine serum albumin.

The chimeric Jag1/F_{c} protein was "clustered" by preincubation with a ten-fold weight excess of goat anti-human F_{c} (Sigma, St. Louis MO). Before use, the antibody solution (2 mg/ml) was dialyzed overnight, with three changes, against PBS to remove sodium azide. The volume of antibody solution was not changed by dialysis. For clustering, Jag1/F_{c} (1 mg/ml in PBS + 0.1% BSA) and anti-human F_{c} (2 mg/ml in PBS) were mixed and incubated at room temperature for 40 min prior to addition to the cell cultures.

Clustered Jag1/F_{c} was added to cultures at a final concentration of 5 µg/ml Jag1/F_{c} and 50 µg/ml antibody. Control cultures received only the antibody. Additional cultures received Jag1/F_{c} and anti-F_{c} as above, along with 5 µM N-[N-(3,5-Difluorophenacetyl)-L-alanyl]-S-phenylglycine t-butyl ester (DAPT), a γ-secretase inhibitor. Stock solutions of 2 mM DAPT in DMSO were obtained from Sigma-Aldrich, St. Louis, MO. γ-secretase is a protease that, upon activation of transmembrane Notch protein by a Notch ligand, cleaves the Notch protein at a site that allows release of the Notch intracellular domain. Certain cultures also included BMP-6 (final concentration 500 ng/ml), as described in Example 5.

After 10 days in culture, cells were fixed in methanol, stained with Alizarin Red and examined by microscopy as described in Example 5. When the endogenous Notch protein was activated in MASCs by soluble Jagged1 ligand, concomitant with osteogenic stimulation, osteogenesis was strongly enhanced. The effect was observed in either the absence or presence of BMP6. Additional experiments showed that stimulation of osteogenesis by Jagged was dose-dependent.

Stimulation of osteogenesis by Jagged is completely reversed by DAPT in the absence of BMP6 and strongly reduced in its presence.

These experiments also confirmed the reduced osteogenic potential of DRCs compared to MASCs, and showed that the small degree of osteogenic potential exhibited by DRCs in the presence of BMP6 is reduced by DAPT.

The stimulation of osteogenic differentiation of MASCs by Jagged, and the reversal of this stimulation by the γ-secretase inhibitor DAPT, suggest that liberation of the NICD in a MASC promotes its differentiation along an osteogenic lineage.

### Example 8: Levels of NICD-encoding DNA and mRNA in DRCs during passage

The observation that release of endogenous NICD in MASCs appears to stimulate their differentiation along an osteogenic pathway (Example 7) seemed at odds with the observation that transfection of MASCs with a nucleic acid encoding an exogenous NICD blocked osteogenic differentiation (Example 5). To obtain additional information, levels of NICD-encoding DNA and RNA in transfected MASCs (*i.e.,* DRCs) were determined. To this end, after preparation of DRCs essentially as described in Example 2, samples were removed at each passage (*i.e.,* after a culture had reached 90% confluence and just prior to trypsinization and replating) for DNA and RNA analysis.

To isolate cellular DNA, approximately 2 x 10⁶ cells were collected by trypsinization. The cells were washed in DPBS (Hyclone, Logan, UT) and collected by centrifugation. Cells were resuspended in 0.2 ml DPBS per 2 x 10⁶ cells and lysed by the addition of Proteinase K (Qiagen, Valencia, CA). DNA was purified from lysed cells using a QiAmp DNA mini kit (Qiagen, Valencia, CA).

Isolated cellular DNA was tested for the presence of exogenous Notch sequences by QPCR using a Roche LightCycler® 480 real-time PCR System. Primer sequences were as follows:
F6: TTGGTCTTACTGACATCCACTTTG (SEQ ID NO:1)
R4: GCTAGCCTATAGTGAGTCGTATT (SEQ ID NO:2)

The probe sequence was:
5'CCCAGTTCAATTACAGCTCTTAAGGCTAGAG-3' (SEQ ID NO:3)

This probe contained the fluorophore 6-FAM at the 5' end and the BHQ1® quencher (Biosearch Technologies, Novato, CA) at the 3' end. Oligonucleotides were obtained from Operon Technologies (Huntsville, AL).

100 ng of cellular DNA was present in each 20 µl amplification reaction. Primer concentrations were 900 nM each and probe concentration was 200 nM. The amplification reaction was conducted in LightCycler® 480 Master Mix (Roche, Mannheim, Germany) with a preincubation of 1 min. at 95°C; followed by 40 cycles of 5 min. at 95°C. The detection format used was mono color hydrolysis probe.

For analysis of NICD mRNA levels, cellular RNA was isolated using a Qiagen RNeasy® Mini Kit according to the manufacturer's instructions (Qiagen, Valencia, CA), followed by treatment with DNase (Ambion, Austin, TX).

Isolated cellular RNA was tested for the presence of exogenous Notch sequences by QRT-PCR using a Roche LightCycler® 480 real-time PCR System. Primer sequences were as follows:
Fl: CACTGGGCAGGTGTCCAC (SEQ ID NO:4)
R2: CCATGGTGGCGCTCGAG (SEQ ID NO:5)

The probe sequence was the same as that used for DNA analysis (SEQ ID NO:3, above).

100 ng of cellular RNA was present in each 20 µl amplification reaction. The F1 primer was present at a final concentration of 100 nM; the R2 primer was present at a final concentration of 900 nM, and the probe was present at a final concentration of 200 nM. Reactions also contained 10 Units M-MuLV reverse transcriptase (New England Biolabs, Ipswich, MA) and 6 Units of RNasin® (Promega, Madison, WI). The reverse transcription/amplification reaction was conducted in LightCycler® 480 Master Mix (Roche, Mannheim, Germany) with a preincubation of 30 min. at 48°C; followed by 45 cycles of 5 min. at 95°C. The detection format used was mono color hydrolysis probe.

The results, shown in Figure 1, indicate that, by the first passage (*i.e.,* following transfection with NICD-encoding sequences, one week of selection and two weeks further culture after removal of the selective agent), cells surviving selection contained less than one copy per cell of the transfected NICD-encoding DNA (based on standards made by serial dilution of pN2 in water), and the amount continued to decrease steadily thereafter (Figure 1A). Similarly, mRNA sequences encoding exogenous NICD decrease continuously through passage (Figure 1B). These results are consistent with the possibility that higher initial concentrations of exogenous NICD, that then decrease over time, are involved in blocking the osteogenic potential of DRCs.

### Example 9: Stable expression of exogenous NICD enhances osteogenic potential of MASCs

A possible interpretation of these results is that continuous presence of the NICD leads to osteogenic differentiation of MASCs; but for conversion of MASCs into DRCs, a more transient expression of the NICD, at an early stage of the conversion process (*i.e.,* as results from a one-week selection period), is required. To test this possibility, MASCs were transfected with NICD-encoding vectors whose expression was stabilized by continuous selection, and their osteogenic potential was assayed as described in Example 5.

Two different NICD-encoding vectors were used in these studies. The pN2 vector is the same vector that was used in the previous experiments. It contains sequences encoding a NICD under the transcriptional control of the CMV promoter, and sequence encoding neomycin phosphotransferase under the transcriptional control of the SV40 early promoter and enhancer. The pNFK vector contains a CMV promoter which drives transcription of a multicistronic transcription unit including sequences encoding the human Notch 1 NICD, a Flag immunotag, 2A peptide sequences and a neomycin phosphotransferase gene. Following transcription and translation, the 2A peptide sequence in the resultant polyprotein is cleaved by cellular proteases to generate equimolar amounts of the NICD and neomycin phosphotransferase.

The pN0 vector was used as a control. It contains sequences encoding neomycin phosphotransferase (NPT) under the transcriptional control of the SV40 early promoter and enhancer, and does not contain sequences encoding a NICD.

MASCs, prepared essentially as described in Example 1, were transfected separately with the vectors described above. Transfections were conducted essentially as described for the pN2 transfections in Example 2. Following transfection, cells were maintained under continuous selection with 100 µg/ml G418 for two passages; each passage requiring growth of cells to 90% confluence (as compared to one week of selection to generate DRCs). This process of selection and propagation took approximately two months. Cells were then transferred into osteogenic differentiation medium (Example 5) containing 100 µg/ml G418 and cultured for a further six days, after which they were fixed, stained and examined as described in Example 5.

Results from the osteogenesis assay indicated that, under continuous selection conditions (in which cells grew more slowly), MASCs stably transfected with the NICD-encoding vectors pNFK and pN2 show much stronger Alizarin Red staining than MASCs stably transfected with a vector encoding only NPT (pN0), in both the presence and absence of 500 ng/ml BMP6. Comparison of cells that had not undergone continuous selection showed that DRCs, which underwent only one week of selection after transfection with a NICD-encoding vector, had a weaker osteogenic potential (as evidenced by Alizarin Red staining) than untransfected MASCs.

To quantitate the extent of osteogenic differentiation, Alizarin Red was extracted from duplicates of the samples analyzed as described above, and levels of extracted Alizarin Red were determined by spectrophotometry. The samples were extracted with 0.5N HCl/5%SDS and the extract was transferred to a fresh microwell for determination of absorbance at 450 nm. Figure 2 shows that Alizarin Red levels (and therefore osteogenic potential) were highest in cells that were stably transfected with pNFK and pN2, and lowest in DRCs (labeled SB623 in the figure) and cells transfected with a vector lacking NICD sequences (pN0).

In a separate experiment, levels of NICD-encoding mRNA transcribed from the exogenous DNA in stably transfected cells were determined. At the time that the various stably-transfected cell cultures were transferred into differentiation medium, separate 0.5 ml samples of cell suspension were plated in 10 cm dishes and cultured for three days. NICD-encoding mRNA in these cell samples were determined as described in Example 8. NICD mRNA levels were normalized to levels of GAPDH mRNA, and the normalized value in DRCs was arbitrarily set to 1.0. Figure 3 shows the results, expressed as levels of GAPDH-normalized NICD mRNA relative to that present in DRCs (labeled "SB" in the Figure). The highest levels of NICD-encoding mRNA were found in the pN2- and pNFK-stably transfected cells. Since these cells also show the highest levels of Alizarin Red staining (e.g., Figure 2), these results are consistent with the idea that high NICD levels at late times after transfection stimulate osteogenic differentiation.

### Example 10: Adipogenic potential of MASCs is inhibited by activation with Jagged, a Notch ligand

This example shows that, although generation of intracellular NICD by activating cells with Jagged1 stimulated the osteogenic potential of MASCs, it inhibited their adipogenic potential.

MASCs were prepared essentially as described in Example 1. Culture in adipogenic differentiation medium was as described in Example 6. Treatment of cells with Anti-F_{c} and Clustered Jag1/F_{c} were as described in Example 7. Fixation, staining with Oil Red O and analysis of adipogenic differentiation were as described in Example 6.

The results of this experiment indicated that little to no adipogenic differentiation was observed in the absence of DAPT or BMP6; though what little adipogenesis did occur was blocked by Jag. DAPT (which inhibits generation of the NICD) slightly enhanced adipogenesis. The presence of BMP6 in the differentiation medium strongly enhanced adipogenesis; and this effect is reversed by Jag. Stimulation of adipogenesis by BMP6 is enhanced further by DAPT; and Jag has no effect in this case. DRCs exhibited little to no adipogenic potential under any of these conditions. These results indicate that Notch activation by Jagged inhibits adipogenesis in MASCs; while blocking generation of the NICD with DAPT stimulates adipogenesis, especially in the presence of BMP6.

### Example 11: Effect of sustained expression of NICD on adipogenesis

In additional experiments, the effect of sustained expression of the NICD on adipogenesis, in stably transfected cell cultures, was determined. The results of these experiments showed that, after continuous selection in G418, no adipogenesis (assayed by Oil Red O staining) was observed in cells stably transfected with pNFK or pN2; while cells stably transfected with pN0 exhibit adipogenesis in the presence of BMP. As shown previously, MASCs are capable of undergoing adipogenic differentiation; while the adipogenic differentiation potential of DRCs is absent or greatly reduced compared to MASCs. These results indicate that that both transient and stable expression of an exogenous NICD results in inhibition of adipogenesis.

### Example 12: Alterations in gene expression in differentiation-restricted cells

To determine whether changes in gene expression accompany the restrictions in developmental potential that result from transfection of MASCs with NICD-encoding sequences, expression of selected genes in MASCs was compared to expression of the same genes in DRCs, by measuring their mRNA levels.

In the first experiment, two direct targets of NICD action, the Hes1 and Hey1 genes, were analyzed. Under normal conditions, activation of membrane-bound Notch results in cleavage of the intracellular domain (NICD), which translocates to the nucleus and binds to an inhibitor of Hes1 and Hey1 transcription. The result is that transcription of the Hes1 and Hey1 genes is activated.

Accordingly, levels of Hes1 and Hey1 mRNAs were measured in several matched sets of DRCs and MASCs (*i.e.,* MASCs were obtained from a donor and a portion of those MASCs were converted to DRCs). Total RNA was purified and used as template for RT-PCR, using PCR primers specific for Hes1 and Hey1. GAPDH mRNA levels were also measured in the same cells, for use as a normalization standard.

As shown in Table 1, Hey 1 mRNA levels were increased in DRCs compared to their parental MASCs, consistent with previous observations following Notch activation *in vivo.* Surprisingly, however, levels of Hes 1 mRNA decreased in DRCs. Without wishing to be bound by theory, the inventor notes that, during the conversion of MASCs to DRCs, selection for the presence of the NICD is conducted for a period of one week. Thus the extent and/or duration of NICD activity may differ from that which occurs during physiological NICD activation, and this may result in different downstream effects compared to those that occurs following normal Notch activation. In addition, the Hes1 gene product negatively autoregulates its expression; thus, initial activation of Hes1 by the NICD in DRCs may be followed by its auto-repression.

**Table 1: Relative mRNA levels of NICD-activated genes in DRCs compared to their parental MASCs***

| **Donor** | **Hes 1** | **Hey 1** |
|---|---|---|
| D33 | 0.09 | |
| D36 | 0.21 | |
| D38 | 0.25 | |
| D41 | 0.55 | 2.11 |
| D51 | 0.74 | 3.52 |
| **Average** | 0.37 | 2.82 |
| **Std. Dev.** | 0.27 | 1.0 |

| | | |
|---|---|---|
| * Hes 1 levels were measured and compared in five different sets of matched DRC/MASC pairs and the ratio of levels in DRCs/levels in MASCs is given. In two of those donor pairs, Hey levels were also measured. Average ratios and standard deviations are presented in the bottom two rows. | | |

### Example 13: Analysis of additional Notch targets, notch pathway members and Wnt pathway members

The expression of a number of genes involved in Notch signaling, and in signaling pathways that interact with the Notch pathway (Wnt, Shh), was compared in DRCs and MASCs, using a commercial array (RT² Profiler PCR Array Human Notch Signaling Pathway plate, SA Biosciences No. PAHS-059F). The genes that were analyzed are shown in Table 2 (taken from SA Biosciences online Catalogue). Notch pathway members include genes encoding Notch ligands, enzymes that modify the Notch protein and various transcriptional regulatory proteins that control the expression of the Notch pathway members. Notch pathway targets are genes whose expression is either directly or indirectly regulated by Notch activation. Wnt pathway members are Wnt ligands or proteins that participate in WNT-mediated signaling pathways. The Wnt pathway was investigated because of known crosstalk between the Notch and Wnt signaling pathways, and because it is involved in osteogenic differentiation, which is inhibited in DRCs.

**Table 2: Genes involved in, or related to, Notch signaling**

| **Notch Signaling Pathway:** |
|---|
| Notch Binding: DLL1 (DELTA1), DTX1, JAG1, JAG2. |
| Notch Receptor Processing: ADAM10, PSENI, PSEN2, PSENEN (PEN2). |

| **Notch Signaling Pathway Target Genes:** |
|---|
| Apoptosis Genes: CDKN1A, CFLAR (CASH), IL2RA, NFKB1. |
| Cell Cycle Regulators: CCND1 (Cyclin D1), CDKN1A (P21), IL2RA. |
| Cell Proliferation: CDKN1A (P21), ERBB2, FOSL1, IL2RA. |
| Genes: Regulating Cell Differentiation: DTX1, PPARG. |
| Neurogenesis: HES1, HEY1_{.} |
| Regulation of Transcription: DTX1, FOS, FOSL1, HES1, HEY1, NFKB1, NFKB2, NR4A2, PPARG, STAT6. |
| Other Target Genes with Unspecified Functions: CD44, CHUK, IFNG, IL17B, KRT1, LOR, MAP2K7, PDPK1, PTCRA. |

| **Other Genes: Involved in the Notch Signaling Pathway:** |
|---|
| Apoptosis Genes: AXIN1, EP300, HDAC1, NOTCH2, PSEN1, PSEN2. |
| Cell Cycle Regulators: AXIN1, CCNE1, CDC16, EP300, FIGF, JAG2, NOTCH2, PCAF. |
| Cell Proliferation: CDC16, FIGF, FZD3, JAG1, JAG2, LRP5, NOTCH2, PCAF, STIL (SIL). |
| Genes Regulating Cell Differentiation: DLL1, JAG1, JAG2, NOTCH1, NOTCH2, NOTCH3, NOTCH4, PAX5, SHH. |
| Neurogenesis: DLL1, EP300, HEYL, JAG1, NEURL, NOTCH2, PAX5, RFNG, ZIC2 (HPE5). |
| Regulation of Transcription: AES, CBL, CTNNB1, EP300, GLI1, HDAC1, HEYL, HOXB4, HR, MYCL1, NCOR2, NOTCH1, NOTCH2, NOTCH3, NOTCH4, PAX5, PCAF, POFUT1, RUNX1, SNW1 (SKIIP), SUFU, TEAD1, TLE1. |
| Others Genes: with Unspecified Functions: ADAM17, GBP2, LFNG, LM02, MFNG, MMP7, NOTCH2NL, NUMB, SEL1L, SH2D1A. |

| **Other Signaling Pathways that Crosstalk with the Notch Signaling Pathway:** |
|---|
| Sonic Hedgehog (Shh) Pathway : GLI1, GSK3B, SHH, SMO, SUFU. |
| Wnt Receptor Signaling Pathway: AES, AXIN1, CTNNB1, FZD1, FZD2, FZD3, FZD4, FZD6, FZD7, GSK3B, LRP5, TLE1, WISP1, WNT11. |
| Other Genes: Involved in the Immune Response: CXCL9, FAS (TNFRSF6), G1P2, GBP1, IFNG, IL2RA, IL2RG, IL4, IL4R, IL6ST, IRF1, ISGF3G, OAS1, OSM, STAT5A, STUB1. |

MASCs and DRCs, prepared and frozen as described in Examples 1-3, were thawed (Example 4), grown for 4-7 days in alpha-MEM/10% FBS/pen-strep, then replated into 10-cm Petri dishes at a concentration of 0.5 x 10⁶ cells/dish, in duplicate. After re-plating, cells were grown for 4 days to allow intracellular levels of endogenous NICD, activated by trypsinization, to return to basal. At the end of this period, culture medium was carefully aspirated, the cells were lysed in 400 µl RLT Buffer (Qiagen, Valencia, CA), and the lysate was scraped from the plate.

As a control, MASCs that had been exposed to clustered soluble Jagged1 protein, to activate the Notch signaling pathway (see Example 7), were also analyzed and compared to MASCs.

The lysate was homogenized using a QiaShredder (Qiagen, Valencia, CA). The homogenate was mixed with ethanol, and the mixture was applied to a RNeasy column (Qiagen, Valencia, CA) and processed according to the manufacturer's instructions. The eluate was treated with a DNA-free™ kit (Ambion, Austin, TX), according to the manufacturer's protocol, for elimination of genomic DNA.

RNA concentration and purity was assayed on Nanodrop spectrophotometer (Thermo Scientific, Wilmington, DE). If an RNA sample had an OD260/OD280 ratio >2 and an OD260/OD230 ratio >1.7, the RNA samples were used for further analysis. If an RNA sample did not meet these criteria, it was re-purified using a RNeasy column (as above) and re-assayed spectrophotometrically. When the requisite purity was obtained, the RNA samples were converted to cDNA using the Superarray RT² First Strand kit (SA Biosciences, Frederick, MD, Cat # C-03), using 0.5 ug of RNA template. First strand synthesis was conducted in parallel for samples of MASCs and DRCs from the same donor.

cDNA samples, synthesized as described above, were diluted and added to RT² SYBR Green qPCR Master Mix (SA Biosciences) according to the manufacturer's recommendations. Samples were then applied to the RT² Profiler PCR Array Human Notch Signaling Pathway plate (SA Biosciences No. PAHS-059F), according to the manufacturer's protocol. The plate contains, in each well, a primer pair specific to a particular gene (see Table 2 for a list of genes that were tested). Real-time PCR was conducted on a Light Cycler R (Roche) according to the manufacturer's instructions and using a program recommended by SA Biosciences for assaying its RT² arrays. As amplification product is produced and self-anneals, SYBR green dye binds to doublestranded material. SYBR green fluorescence, normalized to controls also present on the array, is used a s a measure of mRNA level.

Results were analyzed using analysis tools provided by SA Biosciences, assigning results from MASCs to the "Control" field and DRC results to the "Test" field, to obtain figures for relative changes in expression for the genes assayed. This analysis was conducted for each of three donor pairs of MASCs and DRCs, and the results were averaged.

Table 3 shows a list of genes (out of the 84 tested on the array) whose expression was altered by at least two-fold in Jag-activated MASCs. Jag-activated MASCs were tested to provide results for cells in which Notch activation and NICD release occur through the physiological mechanism of contact between a Notch ligand and cell-surface Notch receptor. In addition, levels of expression of certain genes were compared in DRCs and their progenitor MASCs. The ratios of their mRNA levels in DRCs to their mRNA levels in MASCs (averaged across three pairs of MASCs and DRCs, each pair obtained from the same donor) are given in Table 4.

Criteria for changes in gene expression, for both types of analysis, were established as follows. Any gene whose mRNA levels increased by two-fold or more in DRCs compared to MASCs, or in Jag-activated MASCs compared to MASCs, was scored as activated; and any gene whose mRNA levels decreased by two-fold or more in DRCs or Jag-activated MASCs, compared to MASCs (*i.e.* levels were 50% or less in DRCs or Jag-activated MASCs, compared to MASCs) was scored as repressed.

Using these criteria, 18 genes exhibited activated expression, and three genes showed reduced expression, in Jag-activated MASCs, compared to MASCs. Of these 21 genes, only four genes (HR, Hes1, Hey1 and Wisp1) exhibited changes in their expression, over two-fold in either direction, in DRCs (see Table 4). Expression of Hey1 was activated, and that of HR, Hes1 and Wisp1 was repressed. Interestingly, while expression of HR, Hes1 and Wisp1 was repressed in DRCs; expression of these genes was up-regulated in Jag-activated MASCs. In addition, expression of CCND1 (cyclin D1, a cell-cycle regulator), was activated in DRCs compared to MASCs; while its expression was not altered in Jag-activated MASCs, according to the criteria set forth above.

These changes in gene expression serve to distinguish DRCs from their progenitor cells (MASCs) and can influence their altered developmental potential.

**Table 3: Genes whose expression was altered after activation of the Notch pathway in MASCs by activation of MASCs with clustered Jagged ligand**

| **Abbreviation** | **Name of Gene** | **Additional information** |
|---|---|---|
| DLL1 | Delta-like 1 | Homologue of a *Drosophila* Notch ligand |
| HR | Hairless | Homologue of murine transcriptional repressor |
| JAG1 | Jagged 1 | Homologue of a *Drosophila* Notch ligand |
| LFNG | LFNG-O-fucoslypeptide 3-beta-N-acetylglucosaminy ltransferase | Glycosylates Notch protein |
| NEURL | *Neuralized* homologue | Ubiquitination of Delta/serrate/lag-2 family proteins |
| NOTCH3 | *Notch* homologue 3 | Notch receptor |
| PCAF | p300/CBP-associated factor | Transcriptional regulatory protein |
| CD44 | Cluster of Differentiation 44 | Hematopoietic cell surface marker |
| CDKN1A | Cyclin dependent kinase inhibitor A1 | Cell cycle regulator |
| FOSL1 | *FOS-*like antigen 1 | Transcription factor,; regulates cell proliferation |
| HES1 | Hairy and enhancer of split 1 | Neural differentiation factor |
| HEY1 | Hairy/enhancer of split-related with YRPW motif 1 | Neural differentiation factor |
| MAP2K7 | Mitogen-activated protein kinase 7 | Signal transduction mediator |
| PDPK1 | 3-phosphoinositide-dependent protein kinase 1 | Mediator of growth factor signaling |
| PPARγ | Peroxisome proliferator-activated receptor gamma | Nuclear receptor; transcription factor |
| FZD3 | *Frizzled* homologue 3 | *Wnt* receptor |
| FZD4 | *Frizzled* homologue 4, CD344 | *Wnt* receptor |
| FZD6 | *Frizzled* homologue 6 | *Wnt* receptor |
| WISP1 | *Wnt1* inducible signaling pathway protein 1 | Mediator of *Wnt* signaling |
| WNT 11 | Wingless-type MMTV integration site family member 11 | *Wnt* homologue |
| GLI1 | Glioma-associated oncogene homologue 1 | Zinc finger protein |

**Table 4: Array analysis of gene expression in DRCs compared to MASCs**

| | **Gene^{‡}** | **Ratio DRC/MASC*** | **Std. Dev.** |
|---|---|---|---|
| Notch pathway members | DLL1 | 0.86 | 0.08 |
| | HR | 0.38 | 0.27 |
| | JAG1 | 0.86 | 0.09 |
| | LFNG | 0.57 | 0.19 |
| | NEURL | 1.68 | 1.19 |
| | NOTCH3 | 0.86 | 0.14 |
| | PCAF | 1.32 | 0.79 |
| Notch pathway targets | CCND1 | 2.11 | 0.42 |
| | CD44 | 0.81 | 0.74 |
| | CDKN1A | 1.04 | 0.37 |
| | FOSL1 | 1.07 | 0.09 |
| | HES1 | 0.49 | 0.21 |
| | HEY1 | 2.85 | 1.09 |
| | MAP2K7 | 0.95 | 0.86 |
| | PDPK1 | 1.13 | 0.15 |
| | PPARγ | 0.82 | 0.40 |
| *Wnt* pathway members | FZD3 | 1.74 | 2.44 |
| | FZD4 | 1.51 | 0.5. |
| | FZD6 | 1.10 | 0.29 |
| | WISP1 | 0.40 | 0.11 |
| | WNT11 | 1.96 | 2.22 |

| | | | |
|---|---|---|---|
| ‡ See Table 2 for identification of genes * Averaged from matched MASC/DRC pairs from three donors | | | |

Aspects or embodiments of the invention may also be provided according to the following paragraphs:
**1.** A method for altering the developmental potential of a marrow adherent stromal cell, the method comprising:
   (a) providing a marrow adherent stromal cell; and
   (b) transfecting the cell with a nucleic acid comprising sequences encoding a Notch intracellular domain (NICD).
**2.** The method according to embodiment 1, wherein development of the marrow adherent stromal cell into an osteogenic lineage is reduced.
**3.** The method according to embodiment 1, wherein development of the marrow adherent stromal cell into an adipogenic lineage is reduced.
**4.** The method according to embodiment 1, wherein development of the marrow adherent stromal cell into both osteogenic and adipogenic lineages is reduced.
**5.** The method according to embodiment 1, wherein the NICD is a human NICD.
**6.** The method according to embodiment 5, wherein the NICD comprises amino acids 1703-2504 of the human Notch protein.
**7.** The method of paragraph 1, wherein transfection is transient.
**8.** The method of paragraph 1, wherein transfection is stable.
**9.** The method of paragraph 1, wherein the NICD is expressed transiently.
**10.** The method of paragraph 1, wherein the NICD is stably expressed.

## Claims

1. An in vitro method for altering the developmental potential of a marrow adherent stromal cell (MASC), the method comprising:
(a) providing a marrow adherent stromal cell; and
(b) transfecting the cell with a nucleic acid comprising sequences encoding a Notch intracellular domain (NICD);
wherein the alteration in developmental potential results in differentiation of the cell into a non-mesenchymal lineage.

2. The method of claim 1, wherein said transfection is transient.

3. The method of claim 1, wherein the NICD is a human NICD.

4. The method of claim 1, wherein the non-mesenchymal lineage is a neural lineage.

5. The method of claim 1, wherein the non-mesenchymal lineage is a myogenic lineage.

6. The method of claim 1, wherein gene expression is altered in the cell of non-mesenchymal lineage compared to the MASC.

7. The method of claim 6, wherein expression of the Hey1 and cyclin D1 genes is increased.

8. The method of claim 6, wherein expression of the Hes1, HR and Wisp1 genes is decreased.
